# FASCICULE DE BREVET EUROPEEN

(11) **EP 1 280 420 B1**
(45) Date de publication et mention de la délivrance du brevet: **30.01.2008**
(21) Numéro de dépôt: 01917177.6
(22) Date de dépôt: 19.03.2001
(51) Int. Cl.: A23L 1/30, A23D 9/00, C11B 3/12, A61K 31/23, A23L 1/24, A21D 2/16

(54) **HUILE VEGETALE NATURELLE CONCENTREE EN INSAPONIFIABLE COMME INGREDIENT ALIMENTAIRE**
NATÜRLICHES PFLANZENÖL MIT ANGEREICHERTEM UNVERSEIFBAREM ANTEIL ALS NAHRUNGSMITTELBESTANDTEIL
NATURAL VEGETABLE OIL CONCENTRATED IN UNSAPONIFIABLE MATTERS AS FOOD INGREDIENT

(30) Priorité: 20.03.2000 FR 0003522
(43) Date de publication de la demande: 05.02.2003
(73) Titulaire: Laboratoires Expanscience, 92400 Courbevoie (FR)
(72) Inventeur: KOHLER, Carole, F-75017 Paris (FR); MSIKA, Philippe, F-75018 Paris (FR); PICCIRILLI, Antoine, F-78000 Versailles (FR)
(74) Mandataire: Ahner, Francis
(86) Numéro de dépôt international: PCT/FR2001/000814
(87) Numéro de publication internationale: WO 2001/070046

(56) Documents cités:
- EP-A- 0 785 249
- WO-A-00/49116
- WO-A-80/02100
- WO-A-98/18888
- DE-A- 19 652 522
- FR-A- 2 102 888
- FR-A- 2 653 974
- FR-A- 2 692 783
- FR-A- 2 762 512
- FR-A- 2 787 714
- OOI ET AL: "Recovery of Carotenoids from Palm Oil" JOURNAL OF THE AMERICAN OIL CHEMISTS' SOCIETY,AMERICAN OIL CHEMISTS' SOCIETY. CHAMPAIGN,US, vol. 71, no. 4, avril 1994 (1994-04), pages 423-426, XP002140505 ISSN: 0003-021X

## Description

La présente invention se rapporte à l'utilisation d' une huile végétale naturelle concentrée en sa fraction insaponifiable comme ingrédient alimentaire, pour la préparation d'une composition alimentaire ou d'un complément alimentaire.

Les huiles végétales et les compositions alimentaires ou compléments alimentaires comprenant ces d'huiles végétales concentrées sont utilisées, au sens de la présente invention, aussi bien à des fins diététique, nutritionnelle que cosmétique.

Le problème posé par la présente invention est de proposer un nouvel aliment ou ingrédient alimentaire enrichi en constituants insaponifiables et notamment en vitamine E et en phytostérols, tout en maintenant un apport en acides gras monoinsaturés, et en acides gras polyinsaturés essentiels comparable à l'apport en ces composés des produits de ce type (huiles classiques), cet aliment enrichi permettant ainsi de réduire l'apport calorique global.

Sur le marché on trouve des huiles généralement supplémentées en vitamine E, ainsi que des compositions alimentaires généralement appelées "aliments fonctionnels" supplémentées en phytostérols, ou encore en bêta-carotène.

Cependant, les molécules apportées en tant que supplément : vitamine E, phytostérols, bêta-carotène...sont obtenues par purification chimique à partir d'une source externe telle que les échappées de désodorisation (sous-produits de l'industrie du raffinage des huiles) et le talloil (sous-produit de l'industrie de la pâte à papier).

FR-A-2653974 décrit l'usage comme additif alimentaire d'un concentrat d'huile de soja (7-11 % d'insaponifiables) en combinaison avec un concentrat d'huile d'avocat (25-45% d'insaponifiables).

FR-A-2692783 divulgue des compositions comprenant un concentrat d'huile de sésame (10-20% d'insaponifiables) et un concentrat d'huile de germe de blé ou de soja (14-30% insaponifiables).

FR-A-2762512 décrit un concentrat d'huile de lupin (45-60% d'insaponifiables) ou d'un mélange 30/70 de concentrat d'huile de germe de blé (15-30% d'insaponifiables) et d'huile de lupin, ainsi que leur usage cosmétique, pharmaceutique ou alimentaire.

FR-A-2102888 décrit un procédé de concentration de la partie insaponifiable de diverses huiles, entre autres l'huile de maïs et l'huile de palme. L'huile est concentrée à 25-36% d'insaponifiables, suivie d'une séparation des insaponifiables pour obtenir une fraction pure d'insaponifiables.

WO 98/18888 concerne un procédé de déodorisation d'huiles, ledit procédé ayant l'avantage de ne pas altérer la teneur en carotènes et tocophérols de l'huile

DE 196 52 522 concerne un procédé de fractionnement et de distillation moléculaire conçu pour concentrer les tocophérols et stérols présents dans les distillats issus du procédé de déodorisation d'huiles.

Les inventeurs de la présente demande proposent une huile végétale naturelle choisie parmi l'huile de palme, l'huile de germe de maïs, et l'huile de colza, concentrée en fraction insaponifiable, comme ingrédient alimentaire. Cette huile peut être obtenue selon un procédé original par rapport à l'art antérieur puisqu'il consiste en la concentration des nutriments par un procédé physique et non en l'apport de nutriments d'une source externe obtenus par voie chimique.

Dans le cadre de la présente invention, on entend par "huile de palme", l'huile de palme non obtenue notamment par frigélisation de l'oléine de palme, obtenue notamment par frigélisation de l'huile de palme.

Il a maintenant été trouvé par la demanderesse que l'utilisation de l'huile végétale naturelle concentrée selon la présente invention permet de résoudre ce problème.

L'huile végétale naturelle concentrée selon l'invention présente en outre les avantages suivants : elle permet de diminuer le risque d'athérogénèse, elle présente des propriétés hypocholestérolémiantes, elle agit dans la prévention de certains cancers et des maladies cardio-vasculaires, dans la stimulation de la réponse immunitaire chez les personnes âgées, dans la réduction du risque de cataracte et dans le retard de la progression des maladies neurovégétatives.

Un autre avantage de l'huile végétale naturelle concentrée selon la présente invention est qu'elle peut être utilisée en cosmétique (« food-cosmétique »), plus particulièrement en vue d'améliorer l'aspect cutané, pour hydrater la peau, maintenir en l'état la barrière cutanée et le ciment intercornéocytaire par un apport en acides gras essentiels et en stérols, en vue de prévenir le vieillissement cutané par piégeage des radicaux libres, et en tant qu'agent anti-inflammatoire.

L'objet de la présente invention est l'utilisation d'une huile végétale naturelle, choisie parmi l'huile de palme, l'huile de germe de maïs, et l'huile de colza, concentrée en insaponifiable comme ingrédient alimentaire telle que la teneur en fraction insaponifiable de cette huile est de 3 à 15% m/m. Par huile végétale "naturelle" on entend une huile dont la source végétale n'a pas été modifiée génétiquement.

Par «ingrédient alimentaire» au sens de la présente demande, on entend que l'huile végétale naturelle concentrée en insaponifiable entre soit dans une composition alimentaire soit dans un complément alimentaire. Par ingrédient alimentaire, n'est notamment pas visée "l'huile de table " comme aliment de grande consommation.

Dans le cadre de la présente invention, la composition alimentaire peut prendre toute forme d'aliment contenant habituellement de l'huile, comme par exemple la forme de biscuits, sauce de salade etc... Ladite composition alimentaire peut alors contenir entre 5 et 50% en poids d'huile végétale concentrée selon la présente invention.

Le complément alimentaire peut se présenter sous forme de l'huile telle que directement obtenue par le procédé d'enrichissement en insaponifiable tel que décrit ci-après ou bien sous forme de gélules ou de capsules molles de gélatine ou végétales dans le cadre de la présente invention. Ledit complément alimentaire peut alors contenir de 10 à 100% en poids d'huile végétale concentrée selon la présente invention.

Plus particulièrement, la fraction en insaponifiable de l'huile végétale naturelle concentrée selon la présente invention se compose de 0,3 à 2,5 % de tocophérol et de 1,3 à 8% de phytostérol en poids.

Encore plus particulièrement, ladite fraction insaponifiable de cette huile végétale concentrée contient :
- de 0,2 à 10 % en masse de stérols, alcools triterpéniques et méthyl-stérols,
- de 0,1 à 2 % en masse de tocophérols et tocotriènols totaux,
- de 0,1 à 7% en masse de squalène,
- de 0,1 à 1 % en masse de carotènes.

La présente invention a notamment trait à l'utilisation de l'oléine de palme concentrée en insaponifiable en tant qu'ingrédient alimentaire, caractérisée en ce que la teneur en insaponifiable varie de 4,5 à 5,5 % dont 1 à 2 % de tocotriènols, 0,4 à 0,6 % de tocophérols, 0,1 à 0,3 % de carotènes et 1 à 4 % de phytostérols.

Typiquement, l'oléine de palme concentrée en insaponifiable se compose en moyenne de 95 % d'acides gras sous la forme de triglycérides, de 5 % d'insaponifiable dont 1,5 % de tocotriènols, 0,5 % de tocophérols, 0,2 de carotènes et de 1,5 % de phytostérols.

Cette oléine de palme concentrée en insaponifiable est équivalente substantiellement à l'oléine de palme (source) pour les apports en acide palmitique (C 16:0) et en acide oléique (C 18:1 n-9)

Elle présente le bénéfice nutritionnel d'être une source privilégiée en tocotriènols dont on connaît aujourd'hui leur rôle d'antioxydant. Elle est aussi une source alimentaire concentrée en vitamine E, couvrant l'Apport Journalier Recommandée (A.J.R.)

Par exemple, une dose quotidienne en ladite oléine de palme concentrée en insaponifiable, fixée entre 1 et 3 g, apporte 6 à 25 mg de vitamine E, 200 à 900 Equivalent Rétinol (vitamine A sous forme de pro-vitamine A) et 15 et 45 mg de phytostérols totaux.

L'oléine de palme ainsi concentrée en insaponifiable est une source supplémentaire d'apport en vitamine E et pro-vitamine A; elle ne remplace en aucun cas un aliment existant. Cette oléine de palme concentrée peut être utilisée comme ingrédient alimentaire, à des doses comprises entre 1 et 3 g par jour et par personne, permettant un rééquilibrage au plan nutritionnel des apports en vitamine E, en pro-vitamine A et en phytostérols dans des produits alimentaires divers ou dans des aliments fonctionnels.

En outre, la présente invention est relative à l'utilisation de l'huile de germe de maïs concentrée en insaponifiable en tant qu'ingrédient alimentaire, caractérisée en ce que la teneur en insaponifiable varie de 9 à 11 % dont 6 à 8 % en phytostérols et de 1 à 3 % en tocophérols.

Typiquement, l'huile de germe de maïs concentrée en insaponifiable se compose en moyenne de 90 % d'acide gras sous la forme de triglycérides, de 10 % d'insaponifiables dont 7 % de phystostérols et 2 % de tocophérols.

Sur le plan nutritionnel, l'huile de germe de maïs concentrée en insaponifiable est équivalente substantiellement à l'huile de germe de maïs (source) pour les apports en acide linoléique essentiel (C18:2 n-6) et oléique (C18:1 n-9).

Elle est une source alimentaire concentrée en vitamine E, couvrant l'Apport Journalier Recommandé (A.J.R.). Elle présente aussi le bénéfice nutritionnel d'être une source privilégiée en phytostérols dont on connaît aujourd'hui le rôle hypocholestérolémiant.

Par exemple, une dose quotidienne en ladite huile de germe de maïs concentrée fixée entre 2 et 8 g, apporte 11 à 45 mg de vitamine E et 140 à 560 mg de phytostérols totaux.

L'huile de germe de maïs ainsi concentrée en insaponifiable est une source supplémentaire d'apport en vitamine E et en phytostérols; elle ne remplace en aucun cas un aliment existant.

Cette huile de germe de maïs concentrée peut être utilisée comme ingrédient alimentaire, à des doses comprises entre 2 et 8 g par jour et par personne, permettant un rééquilibrage au plan nutritionnel des apports en vitamine E et en phytostérols dans des produits alimentaires divers ou dans des aliments fonctionnels.

Enfin, la présente invention est relative à l'utilisation de l'huile de colza concentrée en insaponifiable en tant qu'ingrédient alimentaire, car la teneur en insaponifiable varie de 8 à 10 % en insaponifiable, dont 6 à 8 % en phytostérols et 0,5 à 1,5 % en tocophérols.

Typiquement, l'huile de colza concentrée en insaponifiable se compose en moyenne de 91 % d'acide gras sous la forme de triglycérides, de 9 % d'insaponifiable dont 7 % de phytostérols et 1 % de tocophérols.

Sur le plan nutritionnel, l'huile de colza concentrée en insaponifiable est équivalente substantiellement à l'huile de colza (source) pour les apports en acides essentiels linoléiques (C18:2 n-6) et linoléniques (C18:3 n-9).

Elle est une source alimentaire concentrée en vitamine E, couvrant l'Apport Journalier Recommandé (A.J.R.). Elle présente aussi le bénéfice nutritionnel d'être une source privilégiée en phytostérols dont on connaît aujourd'hui le rôle hypocholestérolémiant.

Par exemple, une dose quotidienne en ladite huile de colza concentrée fixée entre 1,5 et 5 g, apporte 3 à 20 mg de vitamine E (3 à 20 mg α-T.E.) et 105 à 350 mg de phytostérols totaux.

L'huile de colza ainsi concentrée en insaponifiable est une source supplémentaire d'apport en vitamine E et en phytostérols; elle ne remplace en aucun cas un aliment existant.

Cette huile de colza concentrée en insaponifiable peut être utilisée comme ingrédient alimentaire, à des teneurs comprises entre 1,5 et 5 g par jour et par personne, permettant un rééquilibrage au plan nutritionnel des apports en vitamine E et en phytostérols dans des produits alimentaires divers ou dans des aliments fonctionnels.

Le procédé de préparation de l'huile naturelle végétale, choisie parmi l'huile de palme, l'huile de germe de maïs, et l'huile de colza, concentrée en insaponifiable selon l'invention , comprend au moins une étape de distillation moléculaire de l'huile végétale avant concentration dans les conditions suivantes :
- température de 200°C à 280°C,
- pression de 10⁻³ à 10⁻² mmHg.

En outre, la présente invention prévoit l'utilisation d'une huile végétale naturelle concentrée en insaponifiable selon l'invention pour la préparation d'un ingrédient alimentaire en vue d'un apport en acides gras monoinsaturés et polyinsaturés essentiels et d'une réduction de l'apport calorique global ainsi qu'en vue d'une amélioration de l'aspect cutané, pour prévenir le vieillissement cutané par piégeage des radicaux libres, et/ou en tant qu'agent anti-inflammatoire et aussi en tant qu'agent hypocholestérolémiant, en tant qu'agent agissant dans la prévention de l'athérogenèse, de cancers, des maladies cardio-vasculaires, en tant qu'agent stimulant la réponse immunitaire chez les personnes âgées, en tant qu'agent réduisant le risque de cataracte ou en tant qu'agent retardant la progression des maladies neurovégétatives.

L'utilisation cosmétique par voie orale pour amélorer l'aspect cutané, prévenir le vieillissement cutané et le photovieillissement, caractérisée en ce qu'elle comprend l'ingestion d'une huile végétale naturelle concentrée selon l'invention fait également partie de l'invention.

La présente invention concerne également l'utilisation dans la préparation d'un ingrédient alimentaire d'une huile végétale naturelle choisie parmi l'huile de palme, l'huile de germe de maïs et l'huile de colza, concentrée en insaponifiable telle que la teneur en fraction insaponifiable de cette huile est de 5 à 12% m/m.

Plus particulièrement, la fraction en insaponifiable de l'huile végétale naturelle concentrée comprise dans l'ingrédient alimentaire selon la présent invention se compose de 0,3 à 2,5 % de tocophérol et de 1,3 à 8% de phytostérols en poids.

Encore plus particulièrement, cet ingrédient alimentaire est tel que ladite fraction insaponifiable de cette huile végétale concentrée contient :
- de 0,2 à 10 % en masse de stérols, alcools triterpéniques et méthyl-stérols,
- de 0,1 à 2 % en masse de tocophérols et tocotriènols totaux.
- de 0,1 à 7% en masse de squalène,
- de 0,1 à 1 % en masse de carotènes.

De préférence, la composition alimentaire, forme sous laquelle peut se trouver l'ingrédient alimentaire contient de 0,5 à 20 % en masse d'une huile végétale naturelle choisie parmi l'huile de palme, l'huile de germe de maïs, et l'huile de colza, concentrée en insaponifiable par rapport à la masse de la composition alimentaire totale.

De manière avantageuse, cet ingrédient alimentaire est utilisé pour améliorer l'aspect cutané et/ou pour prévenir le vieillissement cutané, le photovieillissement, et la photo-immunodépression, par piégeage des radicaux libres, en tant qu'agent anti-inflammatoire, en tant qu'agent hypocholestérolémiant, en tant qu'agent pour la prévention de l'athérogenèse, de cancers et des maladies cardio-vasculaires, en tant qu'agent stimulant la réponse immunitaire chez les personnes âgées, en tant qu'agent réduisant le risque de cataracte ou en tant qu'agent retardant la progression des maladies neurovégétatives.

L'utilisation selon la présente invention peut aussi être de nature cosmétique telle qu'elle comprend l'ingestion d'huile végétale naturelle choisie parmi l'huile de palme, l'huile de germe de maïs, et l'huile de colza, concentrée en insaponifiable comme ingrédient ou l'administration par voie orale d'un ingrédient alimentaire contenant une huile végétale naturelle choisie parmi l'oléine de palme, l'huile de germe de maïs, et l'huile de colza , concentrée en insaponifiable.

La fraction insaponifiable d'une huile végétale alimentaire est définie comme l'ensemble des constituants qui, après saponification (hydrolyse basique), sont très peu solubles dans l'eau et solubles dans les solvants dits « solvants des graisses », comme par exemple les alcanes, les alcanes chlorés et les éthers.

La proportion d'insaponifiable contenue dans un corps gras dépend bien évidemment de l'origine biologique de ce corps gras, des traitements qu'il a pu subir (raffinage), ainsi que de la nature du solvant d'extraction (éther diéthylique, hexane).

De façon générale, la fraction insaponifiable d'une huile végétale peut comprendre les constituants suivants : des stérols et méthyl-stérols, des tocophérols (vitamine E) et tocotriènols, des carotènes (pro-vitamine A), d'autres composés terpéniques (alcools triterpéniques), des cires et alcools gras, des hydrocarbures aliphatiques saturés et insaturés et du squalène.

En moyenne, les huiles végétales naturelles choisies parmi l'huile de palme, l'huile de germe de maïs, et l'huile de colza, concentrées en insaponifiables, présentent des teneurs en insaponifiable comprises entre 0,5 et 1,5 %. Les différents constituants de l'insaponifiable des huiles végétales ont tous des masses moléculaires inférieures à celles des triglycérides. Il est donc possible de séparer ces composés des glycérides, au moins partiellement, afin d'obtenir une huile végétale concentrée en insaponifiable.

L'huile végétale naturelle choisie parmi l'huile de palme, l'huile de germe de maïs, et l'huile de colza, concentrée en insaponifiable selon la présente invention peut avantageusement être obtenue par distillation moléculaire.

La distillation moléculaire (ou distillation à court trajet) est un procédé physique d'évaporation fractionnée effectué sous vide poussé (10⁻² à 10⁻³ mm de Hg) et à haute température (200-300°C). Cette technique est particulièrement adaptée à la distillation des composés thermosensibles, de hauts poids moléculaires et de faible volatilité (phytostérols, vitamines naturelles, huiles et corps gras, arômes...).

Sur un plan pratique, la distillation moléculaire est mise en oeuvre dans des distillateurs de type centrifuge à plateaux tournants ou de type film raclé.

Ce procédé permet notamment de réaliser des opérations de séparation, de purification, de décoloration et de désodorisation. Cette technique est reconnue comme moyen de purification physique utilisable dans l'industrie des arômes et des ingrédients (ex. purification de la vitamine E naturelle).

Appliquée aux huiles végétales, la distillation moléculaire permet d'extraire préférentiellement les composés les plus légers qui les constituent (fraction insaponifiable). En effet, sous un vide poussé et à une température adéquate, la fraction insaponifiable est vaporisée puis recondensée, constituant ainsi le distillat.

Dans le cas de la distillation d'une huile végétale naturelle, le distillat est appelé « huile végétale concentrée en insaponifiable », ce distillat a été concentré en insaponifiable de l'huile source. La teneur en insaponifiable de l'huile végétale concentrée peut atteindre 10 fois la teneur en insaponifiable de l'huile source correspondante.

Cet enrichissement en insaponifiable correspond à une élimination partielle des triglycérides.

Il a en outre été vérifié que ce procédé n'entraînait aucune modification chimique ou altération des composés de l'insaponifiable, et que les fractions fortement insaturées étaient préservées. Par conséquent, la répartition en acides gras de l'huile végétale concentrée est identique à celle de l'huile végétale avant concentration.

De préférence l'huile végétale naturelle concentrée selon la présente invention est obtenue par distillation moléculaire de l'huile végétale correspondante dans les conditions suivantes : température de 200 à 280 °C, pression de 10⁻³ à 10⁻² mmHg.

Comme indiqué précédemment, les propriétés nutritionnelles de l'ingrédient alimentaire selon l'invention se définissent au niveau :
des acides gras constitutifs, notamment l'acide linoléique C18:2 (n-6) et l'acide linolénique C18:3 (n-3), mais aussi l'acide oléique C18:1 (n-9), de sa teneur en vitamine E, de sa teneur en phytostérols, en squalène et carotènes.

### L'acide oléique.

Des études nutritionnelles récentes, réalisées en particulier sur l'huile d'olive (régime méditerranéen), ont montré le rôle de l'acide oléique dans le métabolisme du cholestérol circulant via les lipoprotéines LDL et HDL .
L'acide oléique conduit à une diminution du LDL-cholestérol et conjointement à une augmentation du HDL-cholestérol, d'où un effet de diminution du risque d'athérogenèse. L'acide oléique participe donc à la protection contre les maladies cardio-vasculaires.

Ainsi l'ingrédient alimentaire selon l'invention contribue à un apport en acides gras monoinsaturés, via l'acide oléique.

### L'acide linoléique.

De très nombreuses études nutritionnelles ont été réalisées pour montrer le rôle essentiel de cet acide gras.
L'acide linoléique C18:2 (n-6) est dit acide gras essentiel indispensable, c'est à dire que notre organisme ne sait pas le synthétiser à partir d'autres acides gras. Il est le précurseur (ou chef de file) de la série métabolique des acides gras polyinsaturés dits n-6 ou ω 6, qui sont aussi des acides gras « essentiels » en raison de leurs fonctions vitales pour l'organisme.

Cette série d'acides gras comprend, entre autres, l'acide arachidonique C20:4 qui est à la base de la synthèse des eïcosanoides (prostaglandines, thromboxanes et leucotriènes). Les eïcosanoides sont des molécules à haute activité biologique, dites médiateurs chimiques oxygénés qui interviennent dans le mécanisme de l'agrégation plaquettaire. Les eicosanoïdes interviennent aussi dans le contrôle de l'agrégation plaquettaire (prostaglandine de type I - ou PG I- et thromboxane A -ou TX.A-), le contrôle de la fonction rénale (PG.E, PG.F), les phénomènes inflammatoires et immunitaires (leucotriènes de type B - ou LT.B) ...

L'acide linoléique joue un rôle hypocholestérolémiant.

Outre leur rôle métabolique, l'acide linoléique et les acides gras insaturés de la série (n-6) jouent un rôle structural au sein des membranes cellulaires et interviennent dans le contrôle du « degré d'ordre » (couramment appelé « fluidité ») membranaire, lequel contrôle l'activité de certaines enzymes membranaires, de récepteurs membranaires (par exemple les hormones) etc...

L'acide linoléique est un cas particulier intéressant car il entre dans la composition des acyl-céramides (céramide dont l'acide gras est un acide gras ω-hydroxylé, ce groupement ω-OH étant toujours estérifié par le COOH d'un acide linoléique). Ces acyl-céramides en s'associant à la partie protéique des cornéocytes, jouent un rôle déterminant dans la fonction de barrière de l'épiderme (contrôle de la déperdition d'eau), dans la limitation de la pénétration d'agents agresseurs cutanés. A titre d'exemple, les céramides de type 1 sont déficients dans l'acné, la dermatite atopique et le vieillissement.

Ainsi l'ingrédient alimentaire selon l'invention contribue à l'apport nutritionnel recommandé en acide linoléique indispensable à l'organisme lorsque l'huile végétale naturelle est l'huile de germe de maïs concentrée, et l'huile de colza concentrée.

### Vitamine E.

Les tocophérols présentent une activité vitaminique variable selon leur forme : la forme α (alpha) appelée vitamine E est la plus active notamment pour lutter contre le vieillissement, le photovieillissement et la photo-immunodépression car la plus biodisponible au niveau membranaire, les formes β (bêta) et γ (gamma) ayant une activité vitaminique quasi nulle.

La vitamine E est un antioxydant biologique des acides gras polyinsaturés qui agit principalement au niveau des membranes et des lipoprotéines, par piégeage des radicaux libres. Elle aide à prévenir le vieillissement cutané.

D'autres actions sont citées pour la vitamine E :
- intervention dans la prévention de certains cancers,
- stimulation de la réponse immunitaire chez les personnes âgées,
- réduction du risque de cataracte et retard de la progression des maladies neurovégétatives.

Des études nutritionnelles ont montré qu'il n'y a pas de déficience sévère ou carence en vitamine E dans nos régimes alimentaires mais que dans certains cas, l'apport peut être inférieur à l'apport journalier recommandé (A.J.R), soit 10 mg α-T.E par jour.

Les principales sources alimentaires de vitamine E sont les huiles végétales. Le tableau I permet de comparer l'apport en vitamine E de différentes huiles végétales alimentaires. La plupart des huiles végétales sont naturellement riches en vitamine E, les huiles de tournesol et de germe de blé étant des sources privilégiées.

Pour atteindre l'A.J.R, il suffit de consommer environ 1 g à 1,5 g de l'aliment selon la présente invention ou 5 g d'huile de germe de blé ou 15 g d'huile de tournesol par jour.

**TABLEAU I**

| **Apport comparé en vitamine E de différentes huiles végétales, exprimé en mg d'α-tocophérol équivalent (ou mg de vitamine E) pour 100 kcal** | | |
|---|---|---|
| | **mg vit E (mg α-T.E.) /100g** | **mg vit E (mg α-T.E.) /100 kcal** |
| **Germes de blé** | 135-225 | 15-25 |
| **Tournesol** | 45-90 | 5-10 |
| **Maïs** | 27-45 | 3-5 |
| **Colza** | 13,5-27 | 1,5-3 |
| **Soja** | 9-30 | 1-3 |

### SOURCE : MANUEL DES CORPS GRAS (1992), ED. TEC & DOC - LAVOISIER.

Les huiles végétales étant les sources les plus riches en vitamine E et l'ingrédient alimentaire selon la présente invention étant 10 à 100 fois plus concentré en vitamine E que les huiles végétales de grande consommation : tournesol, colza, soja, maïs, l'ingrédient alimentaire selon la présente invention constitue une source particulièrement riche en vitamine E.

### Les phytostérols.

Les phytostérols sont des constituants végétaux naturels de notre alimentation qui sont présents à un niveau de 0,1 à 0,5 % dans les huiles végétales. L'apport journalier en phytostérols totaux est estimé à moins de 500 mg, soit 200 à 300 mg en moyenne.

Les phytostérols, constituants terpéniques majoritaires des insaponifiables végétaux, ont une structure moléculaire proche du cholestérol avec différentes configurations de la chaîne latérale. Les phytostérols ne sont quasiment pas absorbés par la muqueuse intestinale, ainsi l'absorption du β-sitostérol alimentaire ne représente que 5 % des apports.

C'est depuis les années 50 que l'on connaît l'action hypocholestérolémiante des phytostérols.

L'hypothèse du mécanisme d'action est la suivante : les phytostérols provoquent une diminution de l'absorption du cholestérol par compétition avec ce dernier pour sa solubilisation au sein des micelles de sels biliaires dans l'intestin (jéjunum). Une autre hypothèse proposée est l'action des phytostérols sur certaines enzymes du métabolisme lipidique.

Des études menées sur l'animal ou sur l'homme soutiennent, dans leur grande majorité, l'intérêt des phytostérols dans la prévention de l'hypercholestérolémie ou comme un moyen de contrôler une légère hypercholestérolémie.

Par ailleurs, les phytostérols agissent sur l'excrétion des sels biliaires, ce qui pourrait expliquer leur action dans la prévention des cancers colo-rectaux, cette hypothèse demandant des études complémentaires.

Il est également cité que les phytostérols entraînent une diminution du cholestérol sanguin (LDL) et une légère augmentation de la synthèse et de l'excrétion du cholestérol endogène.
Les phytostérols pourraient être impliqués dans d'autres processus biologiques, en cours d'étude :
- inhibition de la prolifération cellulaire,
- activité oestrogénique,
- activité anti-virale.

Toutes les études nutritionnelles réalisées sur les phytostérols démontrent qu'une supplémentation de 0,8 à 3 g / jour (selon la population et les objectifs visés) de phytostérols diminue le cholestérol total et le LDL-cholesterol. Il n'est pas cité d'effet néfaste significatif et il n'est pas donné de dose maximale de sécurité pour les phytostérols.

Finalement, l'ingrédient alimentaire selon l'invention peut présenter le bénéfice nutritionnel d'un apport en acide linoléique C18:2 (n-6), en acide linolénique C18:3 (n-3) acides gras essentiels indispensables à notre organisme, il est également une source alimentaire d'acide oléique C18:1 (n-9). Sur ce point, l'huile concentrée en insaponifiable utilisée dans le cadre de la présente invention est équivalente substantiellement à l'huile végétale de départ.

L'ingrédient alimentaire selon l'invention présente aussi le bénéfice nutritionnel d'être une source privilégiée en phytostérols dont on reconnaît aujourd'hui le rôle hypocholestérolémiant.

Enfin, l'ingrédient alimentaire selon l'invention est une source alimentaire concentrée en vitamine E qui permet de couvrir l'A.J.R. pour cette vitamine liposoluble.

Ainsi, l'ingrédient alimentaire selon l'invention n'est pas un aliment de grande consommation mais bien un ingrédient alimentaire dont l'impact nutritionnel est une complémentation de notre régime alimentaire en vitamine E et en phytostérols.

### METHODE

L'huile végétale naturelle choisie parmi l'oléine de palme, l'huile de germe de maïs, et l'huile de colza, concentrée en insaponifiable est de préférence préparée selon le mode opératoire suivant.

L'huile végétale naturelle alimentaire choisie parmi l'oléine de palme, l'huile de germe de maïs, et l'huile de colza, issue d'une citerne de stockage en acier inoxydable ou en matériau inerte (ex. émail), stockée sous azote, est pompée vers un dégazeur continu. Cet appareil est un évaporateur couche mince de modèle à film tombant. Cette opération a pour but d'éliminer avant la distillation les éventuelles traces d'eau et de gaz dissous (azote, oxygène et dioxyde de carbone...) dans la matière à traiter.

L'huile dégazée est ensuite pompée vers le distillateur moléculaire. Cet appareil fait appel à une technologie de type centrifuge et présente une capacité maximale de distillation de 180 kg par heure. L'enceinte de distillation est composée des éléments principaux suivants :
- un rotor circulaire conique de 90 cm de diamètre,
- un serpentin à circulation d'eau déminéralisée (condenseur),
- deux gouttières de récupération du résidu et du distillat,
- un système de chauffage par induction,
- une cloche à vide.

L'unité de vide est quant à elle composée d'une pompe à palette (vide primaire) et d'une pompe à diffusion d'huile (vide moléculaire).

Au coeur de l'enceinte sous vide, l'huile préalablement dégazée est distribuée en continu à l'aide d'une ligne d'alimentation au centre du rotor. Grâce à la force centrifuge exercée par le rotor (plateau tournant) l'huile dégazée est uniformément répartie sous la forme d'un film à la surface du rotor.

La surface du plateau tournant est chauffée par induction, permettant ainsi la vaporisation instantanée de la fraction insaponifiable (constituants légers) de l'huile dégazée. Les vapeurs d'insaponifiable sont immédiatement condensées à l'aide d'un serpentin à circulation d'eau sur les parois de la cloche, alors que le résidu est entraîné par une gouttière à la périphérie du rotor.

Enfin, le procédé est maîtrisé pour préserver les qualités nutritionnelles de l'aliment selon l'invention, en particulier les acides gras insaturés, la vitamine E et les stérols.

### EXEMPLES.

### Exemple 1: Préparation des huiles végétales concentrées et leur composition.

Les huiles 1.1 à 1.4 ont été préparées par distillation moléculaire, l'insaponifiable est vaporisé à une température de 230 °C, et sous vide poussé de 10⁻³ mm de Hg. La durée de chauffage au cours de la distillation moléculaire est extrêmement courte de l'ordre de 1/10^{ème} de seconde, évitant ainsi la dégradation thermique de la fraction insaponifiable.

Le distillat enrichi en insaponifiable est refroidi en sortie de distillateur puis stocké sous azote, à l'abri de la lumière.

Le distillat enrichi en insaponifiable peut subir une étape de désodorisation sous vide (3mm de Hg) avec injection de 5% de vapeur à 130°C pendant 2 heures.

Cette étape de désodorisation est du même type que celle réalisée lors du raffinage des huiles végétales alimentaires. Le distillat désodorisé est ensuite séché sous azote à 100°C pendant une heure.

### 1.1. HUILE DE TOURNESOL. (NON-REVENDIQUÉE)

### 1) QUALITE.

Il s'agit d'une huile de tournesol concentrée en insaponifiable par distillation moléculaire dont l'aspect est fluide à température ambiante (léger trouble possible). Sa couleur est jaune pâle et son odeur est relativement faible. Indice d'acide de cette huile est de 6,0 mgKOH/g max et son indice de peroxyde de 10 méq O₂ actif/kg max.

### 2). COMPOSITION.

### A. Acides gras.

| | | |
|---|---|---|
| Acide palmitique | C16 | 5 à 12 % |
| Acide stéarique | C18 | 1 à 10 % |
| Acide oléique | C18' | 14 à 35 % |
| Acide linoléique | C18" | 55 à 75 % |
| Acide linolénique | C18'" | < 0,5 % |
| **Acides gras insaturés TOTAUX** | | **> 70 %** |

| | |
|---|---|
| B. Teneur en insaponifiable | : 5 à 10 g/100g |
| C. Teneur en stérols | : > 3,5 g/100g |
| Composition en stérols | : 40% à 65% de β-sitostérol |
| D. Teneur en tocophérols | : < 1 % de brassicastérol > 800 mg/100g |
| Composition en tocophérols | : > 90% d'α-tocophérol |

### 1.2. HUILE DE COLZA.

### 1). QUALITE.

Il s'agit ici d'une huile de colza concentrée en insaponifiable par distillation moléculaire, dont l'aspect est celui d'une graisse semi-fluide à température ambiante, dont la couleur est jaune clair et dont l'odeur est relativement faible.
L'indice d'acide de cette huile de colza est de 6 mgKOH/g max et son indice de peroxyde de 10 méq O₂ actif/kg max.

### 2). COMPOSITION.

### A. Acides gras.

| | | |
|---|---|---|
| Acide palmitique | C16 | 3 à 8% |
| Acide stéarique | C18 | 0,8 à 2,5% |
| Acide oléique | C18' | 50-70% |
| Acide linoléique | C18" | 15 à 28 % |
| Acide linolénique | C18"' | 6 à 14 % |
| Acide érucique | C22' | < 5 % |
| **Acides gras insaturés TOTAUX** | | **> 75 %** |

| | |
|---|---|
| B. Teneur en insaponifiable | : 7 à 11 g/100g |
| C. Teneur en stérols | : > 5 g/100g |
| Composition en stérols | : 40 à 61 % de β-sitostérol |
| | : > 5 % de brassicastérol |
| D. Teneur en tocophérols | : > 800 mg/100g |
| Composition en tocophérols | : > 30 à 50% de α-tocophérol |

### 1.3. HUILE DE GERME DE MAIS

### 1). QUALITE.

Il s'agit ici d'une huile de germe de maïs concentrée en insaponifiable par distillation moléculaire dont l'aspect est fluide à température ambiante pouvant présenter des cristaux de suspension. Sa couleur est jaune doré et son odeur relativement faible.
L'indice d'acide de l'huile de germe de maïs huile de germe de maïs est de 6,0 mgKOH/g max et son indice de peroxyde est de 10,0 méqO₂ actif/kg max.

### 2). COMPOSITION.

### A. Acides gras.

| | | |
|---|---|---|
| Acide palmitique | C16 | 10 à 20% |
| Acide stéarique | C18 | < 3,3 % |
| Acide oléique | C18:1 | 20,0 à 42,2 % |
| Acide linoléique | C18:2 | 34,0 à 65,6 % |
| Acide α-linolénique | C18:3 | < 2,0 % |
| **Acides gras insaturés TOTAUX** | | **> 70,0 %** |

| | |
|---|---|
| B. Teneur en insaponifiable | : 9 à 15 g/100g |
| C. Teneur en stérols | : > 7 g/100g |
| Composition en stérols | : 55,0 à 66,0 % de β-sitostérol |
| | : < 1 % de brassicastérol |
| D. Teneur en tocophérols | : ≥ 1300 mg/100g |
| Composition en tocophérols | : 68,0 à 89 %de γ-tocophérol |

### 1.4. OLEINE DE PALME.

### 1). QUALITE.

Il s'agit ici d'une oléine de palme concentrée en insaponifiable par distillation moléculaire dont l'aspect est celui d'une graisse à température ambiante de couleur crème et de faible odeur.
L'indice d'acide de cette oléine de palme est de 6,0 mgKOH/max et son indice de peroxyde de 10,0 méqO₂ actif/kg max.

### 2). COMPOSITION.

### A. Acides gras.

| | | |
|---|---|---|
| Acide palmitique | C16 | 39,0 à 47,5% |
| Acide stéarique | C18 | 3,5 à 6,0% |
| Acide oléique | C18 :1 | 36,0 à 44,0% |
| Acide linoléique | C18 :2 | 9,0 à 12,0% |
| Acide α-linolénique | C18 :3 | < 0,5% |
| **Acides gras insaturés TOTAUX** | | **> 50,0%** |

| | |
|---|---|
| B. Teneur en insaponifiable | : 5 à 10g/100g |
| C. Teneur en stérols, alcools triterpéniques et methyl-stérols | : > 2g/100g |
| | |
| Composition en stérols | : 50,0 à 62,0% de β-sitostérol |
| | : < 1 % de brassicastérol |
| D. Teneur en tocophérols et tocotriènols | : > 1 000mg/100g |
| Teneur en tocophérols | : > 300 mg/100g |
| Composition en tocophérols | : > 80% d'α-tocophérol |
| Teneur en tocotriènols | : > 700 mg/100g |
| Composition en tocotriènols | : > 45,0% de γ-tocotriènol |

### Exemple 2 : Exemples d'application alimentaire des huiles végétales concentrées de l'invention.

### 2.1. PREPARATION DE BISCUITS SECS (NON-REVENDIQUÉS) à 2 et 10,6% d'huile de tournesol concentrée en insaponifiable.

### Procédé de sablage.

### A. FORMULE.

| | **Préparation à 2%** | | **Préparation à 10,6%** | |
|---|---|---|---|---|
| Farine | 1 500 | 62,5 | 1 500 | 62,5 |
| Margarine | 207 | 8,6 | 0 | 0 |
| **Huile de tournesol *** | 48 | 2,0 | 255 | 10,6 |
| Sucre | 297 | 12,4 | 297 | 12,4 |
| Poudre de lait | 30 | 1,3 | 30 | 1,3 |
| Sel | 9 | 0,4 | 9 | 0,4 |
| Eau | 255 | 10,6 | 255 | 10,6 |
| Poudre levante | 9 | 0,4 | 9 | 0,4 |
| OEuf entier | 45 | 1,9 | 45 | 1,9 |
| **TOTAL** | **2400** | **100** | **2400** | **100** |

| | | | | |
|---|---|---|---|---|
| (*) : huile de tournesol concentrée de l'exemple 1.1. | | | | |

### B. MODE OPERATOIRE.

L'huile de tournesol concentrée et la margarine sont mis à 20°C dans un bol à pétrin en acier inoxydable, et sont mélangés pendant une minute à la vitesse de 75 tours/min. Le sel, le sucre, l'oeuf, la poudre levante et la farine sont ajoutés ; l'ensemble est mélangé durant 5 minutes à la vitesse de 75 tours/min. On ajoute l'eau à 20°C et l'ensemble est mélangé pendant 3 minutes à la vitesse de 75 tours/min.

La pâte ainsi obtenue est fractionnée en biscuits qui sont déposés ensuite sur une plaque métallique. Les biscuits sont cuits dans un four ventilé à 160°C pendant 11 minutes.

**Le biscuit à 2% d'huile de tournesol concentrée** est enrichi de 20 mg/100g en vitamine E native et de 100 mg/100 g en phytostérols natifs de l'huile de tournesol de départ.

**Le biscuit à 10,6% d'huile de tournesol concentrée** est enrichi de 100 mg/100 g en vitamine E native et de 530 mg/100 g en phytostérols natifs de l'huile de tournesol de départ.

### 2.2. PREPARATION DE BISCUITS SECS à 2 et 10,6% d'huile de colza concentrée en insaponifiable. Procédé de crémage.

### A. FORMULE.

| | **Préparation à 2%** | | **Préparation à 10,6%** | |
|---|---|---|---|---|
| Farine | 1 500 | 62,5 | 1 500 | 62,5 |
| Margarine | 207 | 8,6 | 0 | 0 |
| **Huile de colza*** | 48 | 2,0 | 255 | 10,6 |
| Sucre | 297 | 12,4 | 297 | 12,4 |
| Poudre de lait | 30 | 1,3 | 30 | 1,3 |
| Sel | 9 | 0,4 | 9 | 0,4 |
| Eau | 255 | 10,6 | 255 | 10,6 |
| Poudre levante | 9 | 0,4 | 9 | 0,4 |
| OEuf entier | 45 | 1,9 | 45 | 1,9 |
| **TOTAL** | **2400** | **100** | **2400** | **100** |

| | | | | |
|---|---|---|---|---|
| (*) : huile de colza concentrée de l'exemple 1.2 | | | | |

### B. MODE OPERATOIRE.

L'huile de colza concentrée et la margarine sont mis à 20°C dans un bol à pétrin en acier inoxydable, et sont mélangés pendant une minute à la vitesse de 75 tours/min. Le sel, le sucre, et l'oeuf sont ajoutés ; l'ensemble est mélangé durant une minute à la vitesse de 102 tours/min.

On ajoute de l'eau à 40 °C en deux fois ; l'ensemble est mélangé jusqu'à obtention d'un pommadage homogène à la vitesse de 102 tours/min. la farine et la poudre levante sont ajoutées ; l'ensemble est mélangé durant une minute à la vitesse de 75 tours/min.

La pâte ainsi obtenue est fractionnée en biscuits déposés ensuite sur une plaque métallique. Les biscuits sont cuits dans un four ventilé à 160 °C pendant 11 minutes.

**Le biscuit à 2% d'huile de colza concentrée** est enrichi de 10 mg/100 g en vitamine E native, de 140 mg/100 g en phytostérols natifs de l'huile de colza de départ et de 160 mg/100 g en acide gras linolénique (C18:3 n-3 ou ω3).

**Le biscuit à 10,6% d'huile de colza concentrée** est enrichi de 50 mg/100 g en vitamine E native, de 740 mg/100 g en phytostérols natifs de l'huile de colza de départ et de 850 mg/100 g en acides gras linolénique (C18:3 n-3 ou ω3).

### 2.3. PREPARATION D'UNE SAUCE SALADE (NON-REVENDIQUÉE) à 2 et 10% d'huile de tournesol concentrée en insaponifiable. Procédé sous vide pasteurisé.

### A. FORMULE.

| | **Préparation à 2%** | | **Préparation à 10%** | |
|---|---|---|---|---|
| Huile végétale | 480 | 48 | 400 | 40 |
| **Huile de tournesol *** | 20 | 2 | 100 | 10 |
| Protéine sérique | 10 | 1 | 10 | 1 |
| Vinaigre d'alccol | 55 | 5,5 | 55 | 5,5 |
| Moutarde | 40 | 4 | 40 | 4 |
| Poivre | 1 | 0,1 | 1 | 0,1 |
| Sel | 10 | 1 | 10 | 1 |
| Sorbate | 1 | 0,1 | 1 | 0,1 |
| Amidon | 20 | 2 | 20 | 2 |
| Xanthane-Guar | 2,5 | 0,25 | 2,5 | 0,25 |
| Eau | 360,5 | 36,05 | 360,5 | 36,05 |
| **TOTAL** | **1 000** | **100** | **1 000** | **100** |

| | | | | |
|---|---|---|---|---|
| (*) : huile de tournesol concentrée de l'exemple 1.1. | | | | |

### B. MODE OPERATOIRE.

Les poudres (protéine sérique, poivre, sel, sorbate, amidon, xanthane-Guar) sont introduites dans un stephan en acier inoxydable ; l'ensemble est mélangé avec l'eau pendant une minute à 1 500 tours/min. sous vide.

Le concentré d'huile de tournesol et l'huile de tournesol sont introduits en 4 fois à la préparation, à raison d'une dose toutes les minutes. Le vinaigre et la moutarde sont introduits ; l'ensemble est mélangé pendant 20 secondes.

La sauce salade est conditionnée hermétiquement et pasteurisée dns un bain-marie pendant 2 heures à 80°C.

**La sauce salade à 2% d'huile de tournesol concentrée** est enrichie de 20 mg/100 g en vitamine E native et de 100 mg/100 g en phytostérols natifs de l'huile de tournesol de départ.

**La sauce salade à 10% d'huile de tournesol concentrée** est enrichie de 100 mg/100 g en vitamine E native et de 500 mg /100 g en phytostérols natifs de l'huile de tournesol de départ.

### 2.4. PREPARATION D'UNE SAUCE SALADE à 2 et 10% de concentré d'huile de colza Procédé sous air pasteurisé ou stérilisé ou surgelé.

### A. FORMULE.

| | **Préparation à 2%** | | **Préparation à 10%** | |
|---|---|---|---|---|
| Huile végétale | 480 | 48 | 400 | 40 |
| **Huile de colza*** | 20 | 2 | 100 | 10 |
| Protéine sérique | 10 | 1 | 10 | 1 |
| Vinaigre d'alccol | 55 | 5,5 | 55 | 5,5 |
| Moutarde | 40 | 4 | 40 | 4 |
| Poivre | 1 | 0,1 | 1 | 0,1 |
| Sel | 10 | 1 | 10 | 1 |
| Sorbate | 1 | 0,1 | 1 | 0,1 |
| Amidon prégel 126 16 | 20 | 2 | 20 | 2 |
| Xanthane-Guar CX 391 | 2,5 | 0,25 | 2,5 | 0,25 |
| Eau | 360,5 | 36,05 | 360,5 | 36,05 |
| **TOTAL** | **1 000** | **100** | **1 000** | **100** |

| | | | | |
|---|---|---|---|---|
| (*) : huile de tournesol concentrée de l'exemple 1.1. | | | | |

### B. MODE OPERATOIRE.

Les poudres (protéine sérique, poivre, sel, sorbate, amidon prégel, xanthane-Guar) sont introduites dans un stephan en acier inoxydable ; l'ensemble est mélangé avec l'eau pendant une minute à 1 500 tours/min sous-air.
L'huile de colza concentrée et l'huile végétale sont introduits en 4 fois à la préparation, à raison d'une dose toutes les minutes. Le vinaigre et la moutarde sont introduits ; l'ensemble est mélangé pendant 20 secondes.

La sauce salade est conditionnée puis pasteurisée dans un bain-marie pendant 2 heures à 80°C ou stérilisée dans une autoclave rotative pendant 40 minutes à 112°C ou surgelée dans une armoire de congélation à -18°C.

**La sauce salade à 2% d'huile de colza concentrée** est enrichie de 10 mg/100 g en vitamine E native et de 140 mg/100 g en phytostérols natifs de l'huile de colza de départ et de 160 mg/100 g en acide gras linolénique (C18:3 n-3 ou ω3).

**La sauce salade à 10% d'huile de colza concentrée** est enrichie de 50 mg/100 g en vitamine E native et de 700 mg /100 g en phytostérols natifs de l'huile de colza de départ et de 800 mg /100 g en acide gras linolénique (C18:3 n-3 ou ω3).

Les huiles végétales concentrées selon la présente invention, utilisées en tant qu'ingrédient alimentaire ainsi que les compositions et compléments alimentaires contenant cette huile végétale concentrée selon l'invention, possèdent des propriétés hypocholestérolémiantes, agissent dans la prévention de certains cancers et des maladies cardio-vasculaires, dans la stimulation de la réponse immunitaire chez les personnes âgées, dans la réduction du risque de cataracte et dans le retard de la progression des maladies neurovégétatives, ainsi que dans la prévention de du vieillissement cutané, photovieillissement et de la photo-immunodépression.

## Revendications

1. Utilisation d'une huile végétale naturelle choisie parmi l'huile de palme, l'huile de germe de maïs, et l'huile de colza, concentrée en sa fraction insaponifiable, comme ingrédient alimentaire, dans lequel la teneur en fraction insaponifiable de l'huile est de 3 à 15 % m/m.

2. Utilisation selon la revendication 1, **caractérisée en ce que** la fraction insaponifiable se compose de 0,3 à 2,5 % de tocophérol et de 1,3 à 8 % de phytostérol, les pourcentages se référant à la masse totale de l'huile.

3. Utilisation selon l'une des revendications 1 à 2, **caractérisée en ce que** ladite fraction insaponifiable de l'huile végétale concentrée contient :
- de 0,2 à 10 % en masse de stérols, alcools triterpéniques et méthyl-stérols,
- de 0,1 à 2 % en masse de tocophérols et tocotriénols totaux,
- de 0,1 à 7% en masse de squalène,
- de 0,1 à 1 % en masse de carotènes,
les pourcentages se référant à la masse totale de l'huile.

4. Utilisation selon l'une quelconque des revendications 1 à 3, **caractérisée en ce que** la teneur en insaponifiable varie de 4,5 à 5,5 %, ladite fraction insaponifiable contenant 1 à 2 % de tocotriénols, 0,4 à 0,6 % de tocophérols, 0,1 à 0,3 % de carotènes et de 1 à 4 % de phytostérols, les pourcentages se référant à la masse totale de l'huile.

5. Utilisation selon la revendication 1, **caractérisée en ce que** l'huile de germe de maïs concentrée en sa fraction insaponifiable présente une teneur en insaponifiable de 9 à 11 %, ladite fraction insaponifiable contenant 6 à 8 % en phytostérols et de 1 à 3 % en tocophérols, les pourcentages se référant à la masse totale de l'huile.

6. Utilisation selon la revendication 1, **caractérisée en ce que** l'huile de colza concentrée en sa fraction insaponifiable présente une teneur en insaponifiable de 8 à 10 %, ladite fraction insaponifiable contenant 6 à 8 % en phytostérols et 0,5 à 1,5 % en tocophérols, les pourcentages se référant à la masse totale de l'huile.

7. Utilisation selon l'une quelconque des revendications 1 à 6, **caractérisée en ce que** l'huile est préparée selon un procédé comprenant au moins une étape de distillation moléculaire de l'huile végétale dans les conditions suivantes :
- température de 200 à 280 °C.
- pression de 10⁻³ à 10⁻² mmHg.

8. Utilisation selon l'une quelconque des revendications 1 à 6 en vue d'un apport en acides gras monoinsaturés et polyinsaturés essentiels et d'une réduction de l'apport calorique.

9. Utilisation selon l'une quelconque des revendications 1 à 6 pour améliorer l'aspect cutané, prévenir le vieillissement cutané, ou le photovieillissement.

10. Utilisation d'une huile végétale naturelle telle que définie dans l'une quelconque des revendications 1 à 6 pour la préparation d'un ingrédient alimentaire destiné à agir comme agent anti-inflammatoire, à prévenir la photo-immunodépression par piégeage des radicaux libres, à agir comme agent hypocholestérolémiant, agent pour la prévention de cancers et des maladies cardio-vasculaires, agent stimulant la réponse immunitaire chez les personnes âgées, agent réduisant le risque de cataracte ou agent retardant la progression des maladies neurovégétatives.

11. Utilisation selon l'une quelconque des revendications 1 à 6, pour la préparation d'une composition alimentaire ou d'un complément alimentaire.

12. Utilisation selon la revendication 11, **caractérisée en ce que** la composition alimentaire contient de 0,5 à 20% en masse d'huile végétale naturelle concentrée en insaponifiable.

13. Utilisation selon la revendication 11, **caractérisée en ce que** le complément alimentaire contient de 10 à 100% en masse d'huile végétale concentrée en insaponifiable.

14. Utilisation selon l'une quelconque des revendications 11 à 13, **caractérisée en ce que** la composition alimentaire ou le complément alimentaire est destiné(e) à améliorer l'aspect cutané, prévenir le vieillissement cutané, ou le photovieillissement.

15. Utilisation selon l'une quelconque des revendications 11 à 13, **caractérisée en ce que** la composition alimentaire ou le complément alimentaire est destiné(e) à agir comme agent anti-inflammatoire, à prévenir la photo-immunodépression par piégeage des radicaux libres, ou à agir en tant qu'agent hypocholestérolémiant, pour la prévention de cancers et des maladies cardio-vasculaires, agent stimulant la réponse immunitaire chez les personnes âgées, agent réduisant le risque de cataracte ou en tant qu'agent retardant la progression des maladies neurovégétatives.

16. Utilisation selon l'une quelconque des revendications 11 à 13, **caractérisée en ce que** la composition alimentaire ou le complément alimentaire est destiné(e) à un apport en acides gras monoinsaturés et polyinsaturés essentiels et une réduction de l'apport calorique.

## Claims

1. Use of a natural plant oil chosen from palm oil, corn germ oil and rapeseed oil, concentrated in its unsaponifiable fraction, as a food ingredient in which the content of unsaponifiable fraction in the oil is 3% to 15% m/m.

2. Use according to Claim 1, **characterized in that** the unsaponifiable fraction is composed of 0.3% to 2.5% tocopherol and 1.3% to 8% phytosterol, the percentages referring to the total mass of the oil.

3. Use according to either of Claims 1 and 2, **characterized in that** said unsaponifiable fraction in the concentrated plant oil contains:
- from 0.2% to 10% by mass of sterols, triterpene alcohols and methylsterols,
- from 0.1% to 2% by mass of total tocopherols and tocotrienols,
- from 0.1% to 7% by mass of squalene,
- from 0.1% to 1% by mass of carotenes,
the percentages referring to the total mass of the oil.

4. Use according to any one of Claims 1 to 3, **characterized in that** the content of unsaponifiable material ranges from 4.5% to 5.5%, said unsaponifiable fraction containing 1% to 2% tocotrienols, 0.4% to 0.6% tocopherols, 0.1% to 0.3% carotenes and 1% to 4% phytosterols, the percentages referring to the total mass of the oil.

5. Use according to Claim 1, **characterized in that** the corn germ oil concentrated in its unsaponifiable fraction has a content of unsaponifiable material of 9% to 11%, said unsaponifiable fraction containing 6% to 8% phytosterols and 1% to 3% tocopherols, the percentages referring to the total mass of the oil.

6. Use according to Claim 1, **characterized in that** the rapeseed oil concentrated in its unsaponifiable fraction has a content of unsaponifiable material of 8% to 10%, said unsaponifiable fraction containing 6% to 8% phytosterols and 0.5% to 1.5% tocopherols, the percentages referring to the total mass of the oil.

7. Use according to any one of Claims 1 to 6, **characterized in that** the oil is prepared by a process comprising at least one step of molecular distillation of the plant oil under the following conditions:
- temperature from 200 to 280°C,
- pressure from 10⁻³ to 10⁻² mmHg.

8. Use according to any one of Claims 1 to 6, for supplying essential monounsaturated and polyunsaturated fatty acids and for reducing the calorific intake.

9. Use according to any one of Claims 1 to 6, to improve the appearance of the skin, to prevent ageing of the skin or photoageing.

10. Use of a natural plant oil as defined in any one of Claims 1 to 6, for the preparation of a food ingredient intended to act as an anti-inflammatory agent, to prevent photoimmunosuppression by trapping free radicals, to act as a hypocholesterolemiant agent, an agent for preventing cancers and cardiovascular diseases, an agent for stimulating the immune response in the elderly, an agent for reducing the risk of cataracts or an agent for retarding the progress of neurovegetative diseases.

11. Use according to any one of Claims 1 to 6, for the preparation of a food composition or of a food supplement.

12. Use according to Claim 11, **characterized in that** the food composition contains from 0.5% to 20% by mass of a natural plant oil concentrated in unsaponifiable material.

13. Use according to Claim 11, **characterized in that** the food supplement contains from 10% to 100% by mass of a plant oil concentrated in unsaponifiable material.

14. Use according to any one of Claims 11 to 13, **characterized in that** the food composition or the food supplement is intended to improve the appearance of the skin, to prevent ageing of the skin or photoageing.

15. Use according to any one of claims 11 to 13, **characterized in that** the food composition or the food supplement is intended to act as an anti-inflammatory agent, to prevent photoimmunosuppression by trapping free radicals, or to act as a hypocholesterolemiant, for preventing cancers and cardiovascular diseases, as an agent for stimulating the immune response in the elderly, as an agent for reducing the risk of cataracts or as an agent for retarding the progress of neurovegetative diseases.

16. Use according to any one of Claims 11 to 13, **characterized in that** the food composition or the food supplement is intended for supplying essential monounsaturated and polyunsaturated fatty acids and for reducing the calorific intake.

## Patentansprüche

1. Verwendung eines natürlichen pflanzlichen Öls, ausgewählt unter Palmöl, Maiskeimöl und Rapsöl, welches hinsichtlich seines unverseifbaren Anteils angereichert ist, als Nahrungsmittelbestandteil, in welchem der Gehalt an unverseifbarem Anteil 3 bis 15% (Masse/Masse) beträgt.

2. Verwendung nach Anspruch 1, **dadurch gekennzeichnet, dass** der unverseifbare Anteil 0,3 bis 2,5% Tocopherol und 1,3 bis 8% Phytosterol umfasst, wobei die Prozentangaben sich auf die gesamte Masse des Öls beziehen.

3. Verwendung nach einem der Ansprüche 1 bis 2, **dadurch gekennzeichnet, dass** der unverseifbare Anteil des angereicherten pflanzlichen Öls enthält:
- 0,2 bis 10 Massenprozent Sterole, triterpenische Alkohole und Methylsterole,
- 0,1 bis 2 Massenprozent Tocopherole und gesamte Tocotrienole,
- 0,1 bis 7 Massenprozent Squalen,
- 0,1 bis 1 Massenprozent Karotine,
wobei die Prozentangaben sich auf die gesamte Masse des Öls beziehen.

4. Verwendung nach einem der Ansprüche 1 bis 3, **dadurch gekennzeichnet, dass** der Gehalt an unverseifbarem Anteil von 4,5 bis 5,5% variiert, wobei der unverseifbare Anteil 1 bis 2% Tocotrienole, 0,4 bis 0,6% Tocopherole, 0,1 bis 0,3% Karotine und 1 bis 4% Phytosterole enthält, wobei die Prozentangaben sich auf die gesamte Masse des Öls beziehen.

5. Verwendung nach Anspruch 1, **dadurch gekennzeichnet, dass** das an seinem unverseifbaren Anteil angereicherte Maiskeimöl einen Gehalt an unverseifbarem Anteil von 9 bis 11 % aufweist, wobei der unverseifbare Anteil 6 bis 8% an Phytosterolen und 1 bis 3% an Tocopherolen enthält, wobei die Prozentangaben sich auf die gesamte Masse des Öls beziehen.

6. Verwendung nach Anspruch 1, **dadurch gekennzeichnet, dass** das an seinem unverseifbaren Anteil angereicherte Rapsöl einen Gehalt an unverseifbarem Anteil von 8 bis 10% aufweist, wobei der unverseifbare Anteil 6 bis 8% an Phytosterolen und 0,5 bis 1,5% an Tocopherolen enthält, wobei die Prozentangaben sich auf die gesamte Masse des Öls beziehen.

7. Verwendung nach einem der Ansprüche 1 bis 6, **dadurch gekennzeichnet, dass** das Öl hergestellt wird gemäß einem Verfahren, welches wenigstens einen Schritt einer Molekulardestillation des pflanzlichen Öls unter den folgenden Bedingungen umfasst:
- Temperatur von 200 bis 280°C,
- Druck von 10⁻³ bis 10⁻² mmHg.

8. Verwendung nach einem der Ansprüche 1 bis 6 in Hinblick auf eine Zufuhr von einfach ungesättigten und mehrfach ungesättigten essentiellen Fettsäuren und eine Verringerung der Kalorienzufuhr.

9. Verwendung nach einem der Ansprüche 1 bis 6, um das Aussehen der Haut zu verbessern, die Alterung der Haut oder die Photoalterung zu verhüten.

10. Verwendung eines natürlichen pflanzlichen Öls, wie in einem der Ansprüche 1 bis 6 definiert, für die Herstellung eines Nahrungsmittelbestandteils, welcher dazu bestimmt ist, als entzündungshemmendes Mittel zu wirken, die Photoimmundepression durch Einfang der freien Radikale zu verhüten, als hypocholesterinämisches Mittel, Mittel für die Verhütung von Krebserkrankungen und Herz-Kreislauf-Erkrankungen, Mittel, welches die Immunantwort bei älteren Menschen stimuliert, Mittel, welches das Kataraktrisiko verringert, oder Mittel, welches das Fortschreiten von neurovegetativen Erkrankungen verzögert, zu wirken.

11. Verwendung nach einem der Ansprüche 1 bis 6 für die Herstellung einer Nahrungsmittelzusammensetzung oder eines Nahrungsergänzungsmittels.

12. Verwendung nach Anspruch 11, **dadurch gekennzeichnet, dass** die Nahrungsmittelzusammensetzung 0,5 bis 20 Massenprozent natürliches pflanzliches Öl, welches hinsichtlich des unverseifbaren Anteils angereichert ist, enthält.

13. Verwendung nach Anspruch 11, **dadurch gekennzeichnet, dass** das Nahrungsergänzungsmittel 10 bis 100 Massenprozent pflanzliches Öl, welches hinsichtlich des unverseifbaren Anteils angereichert ist, enthält.

14. Verwendung nach einem der Ansprüche 11 bis 13, **dadurch gekennzeichnet, dass** die Nahrungsmittelzusammensetzung oder das Nahrungsergänzungsmittel dazu bestimmt ist, das Aussehen der Haut zu verbessern, die Hautalterung oder die Photoalterung zu verhüten.

15. Verwendung nach einem der Ansprüche 11 bis 13, **dadurch gekennzeichnet, dass** die Nahrungsmittelzusammensetzung oder das Nahrungsergänzungsmittel dazu bestimmt ist, als entzündungshemmendes Mittel zu wirken, die Photoimmundepression durch Einfang der freien Radikale zu verhüten oder als hypocholesterinämisches Mittel, Mittel für die Verhütung von Krebserkrankungen und Herz-Kreislauf-Erkrankungen, Mittel, welches die Immunantwort bei älteren Menschen stimuliert, Mittel, welches das Kataraktrisiko verringert, oder als Mittel, welches das Fortschreiten von neurovegetativen Erkrankungen verzögert, zu wirken.

16. Verwendung nach einem der Ansprüche 11 bis 13, **dadurch gekennzeichnet, dass** die Nahrungsmittelzusammensetzung oder das Nahrungsergänzungsmittel für eine Zufuhr von einfach ungesättigten und mehrfach ungesättigten essentiellen Fettsäuren und eine Verringerung der Kalorienzufuhr bestimmt ist.
